# EUROPÄISCHE PATENTANMELDUNG

(11) **EP 1 235 093 A2**
(43) Veröffentlichungstag der Anmeldung: **28.08.2002**
(21) Anmeldenummer: 02003684.4
(22) Anmeldetag: 18.02.2002
(51) Int. Cl.: G02B 21/00, G02B 5/20, A61B 3/00

(54) **Optische Betrachtungseinrichtung mit Vorrichtung zur partiellen Reduktion der Intensität der Beleuchtung**

(30) Priorität: 21.02.2001 DE 10108254
(71) Anmelder: Leica Microsystems AG, 9435 Heerbrugg (CH)
(72) Erfinder: Bees, Bryan, Downend, GB D Bristol BS162TS (GB)
(74) Vertreter: Rosenich, Paul

(57) **Zusammenfassung**

Die Erfindung betrifft eine optische Betrachtungseinrichtung mit Vorrichtung zur partiellen Reduktion der Intensität der Beleuchtung, beispielsweise ein (Stereo-) Operationsmikroskop. Durch einen Spektralfilter (4), angeordnet im Beleuchtungs-Strahlengang (2), wird die Beleuchtungs-Intensität auf dem Objekt (x,y), beispielsweise einem Auge, reduziert. Die Reduktion erfolgt erfindungsgemäß intensitäts- und/oder wellenlängenabhängig.

## Beschreibung

Die Erfindung betrifft eine optische Betrachtungseinrichtung mit einer Vorrichtung zur partiellen Reduktion der Intensität der Beleuchtung, beispielsweise ein (Stereo-) Operationsmikroskop mit einem Eclipse-Filter. Unter einem Eclipse-Filter versteht man einen Spektralfilter im Beleuchtungs-Strahlengang, welcher die Beleuchtungs-Intensität partiell in einem bestimmten Lichtwellenbereich reduziert.

Das Reduzieren der Beleuchtung in einem bestimmten räumlichen Objekt-Bereich mittels einer ein- bzw. ausklappbaren Blende im Beleuchtungs-Strahlengang wird bei verschiedenen Anwendungen mehr und mehr angewandt, da sich dadurch lichtempfindliche Flächen des Objektes vor zu starker Strahlung schützen lassen.

Diese Blenden werden in der Regel als ein- bzw. ausklappbare Blenden mit vollständiger und/oder teilweisen Abdunkelung des Beleuchtungs-Strahlenganges in einem bestimmten räumlichen Bereich ausgebildet.

In DE 93 01 448 U wird ein Filtersystem mit einzelnen, unterschiedliche Durchmesser aufweisenden Filtern offenbart, bei welchem in bestimmten räumlichen Bereichen eine partielle Abdunkelung möglich ist. Diese ist im sichtbaren Lichtbereich zwischen 400 und 700 nm nicht wellenlängenabhängig ausgestaltet. Es wird ausschließlich darauf verwiesen, dass eine stärkere Abdunkelung im Wellenlängenbereich unter 400 nm stattfinden kann.

Aus DE 88 08 871 U ist ein Diffusionsplättchen für Spaltlampengeräte bekannt, welches eine Vorzugsrichtung in der Streucharakteristik bewirkt.

Darüber hinaus wird in der US 4 715 704 wird von eine Lichtfalle mit zentral abgedunkeltem Bereich beschrieben.

Der Erfinder erkannte, dass die bekannten Systeme nachteilig sind in Bezug auf die folgenden Punkte:
a) Die Reduktion der Lichtintensität erfolgt entweder digital, d.h. der lichtreduzierte Objekt-Bereich ist für den Betrachter entweder dunkel oder in voller Helligkeit sichtbar, oder es gibt zwar eine gestufte Filterwirkung, die jedoch nicht optimiert ist hinsichtlich der Lichtfarbe; dies kann somit negative Auswirkungen auf ein Objekt haben, beispielsweise auf das Auge eines Patienten.
b) Um den lichtreduzierten Bereich für den Betrachter farbecht sichtbar zu machen, ist ein Ausschalten der Blende notwendig, welches für das Objekt eine volle und in der Regel objekt-schädigende Ausleuchtung bedeutet.
c) Durch die fixe Positionierung der Blende im Beleuchtungs-Strahlengang kann die Form und/oder der abgeschwächte Beleuchtungs-Strahlengang nicht variiert werden.
d) Die Schädigungen des Objektes, beispielsweise des Patienten-Auges, erfolgen mit gleicher Gefährlichkeit in der Regel nicht über den gesamten Lichtwellen-Bereich, sondern nur in einem bestimmten Wellenlängenbereich, beispielsweise von 420 nm bis 470 nm und unterhalb von 400 nm.

Der Erfindung liegt somit die Aufgabe zu Grunde, eine Vorrichtung zu schaffen, welche den objekt-empfindlichen Bereich, beispielsweise das Patienten-Auge, optimal schützt und die angegebenen Nachteile vermeidet; und zwar unabhängig davon,
- welche räumliche Form der objekt-empfindliche Bereich aufweist und
- durch welche Intensität der zu schützende Bereich des Objektes verletzt wird.

Gelöst wird diese Aufgabe durch das Einsetzen eines Spektralfilters im Beleuchtungs-Strahlengang, welcher seine spektrale Filterwirkung stufenweise und/oder stufenlos variiert, beispielsweise in Abhängigkeit des Abstandes zur Mittelachse des Filters, sowie
i) vorgegeben räumlich ausgestaltet ist, beispielsweise die Form des zu schützenden Objektes aufweist,
   und/oder
ii) dessen Absorption und/oder Reflexion erfindungsgemäß beliebig zwischen beispielsweise 0% und 90% festgelegt, insbesondere im Lichtwellenlängen-Bereich zwischen 420 und 470 nm, ist
   und/oder
iii) dessen Absorption und/oder Reflexion erfindungsgemäß mittels dünner Schichten wellenlängenabhängig ausgestaltet ist, beispielsweise insbesondere im Lichtwellenlängenbereich zwischen 420 und 470 nm,
   und/oder
iv) in seiner Anwendungsebene und/oder Anwendungsachse und/oder Ausdehnung räumlich verschiebbar ist
   und/oder
v) in Felder mit erfindungsgemäß unterschiedlichen Absorptions- und/oder Reflexions-Eigenschaften gemäß ii) aufteilbar ist, welche stufenförmige bzw. stufenlose Übergänge aufweisen,
   und/oder
vi) in einer speziellen Weiterentwicklungsform beispielsweise als LCD und/oder als elektrochrome Schicht ausbildbar und damit elektronisch ansteuerbar ist.

Durch den vorgängig beschriebenen Einsatz eines Spektralfilters im Beleuchtungs-Strahlengang einer optischen Betrachtungseinrichtung werden in folgenden Schritten die nachstehenden Verbesserungen erreicht:
- Durch die lichtwellenlängen-abhängige Abdunkelung des zu schützenden Objekt-Bereiches wird mittels optimierter Lichtfarben das Objekt vor Verletzungen geschützt.
- Durch die unvollständige Abdunkelung des zu schützenden Objekt-Bereiches erfolgt die Abdunkelung nicht digital, sondern beispielsweise von 0% auf 50%. Dadurch bleibt der abgedunkelte Bereich für den Betrachter in einem bestimmten ungefährlichen Licht-Wellenlängenbereich dauernd sichtbar.
- Wird die zu schützende Objektfläche bis zu einer bestimmten Lichtintensität nicht verletzt, so wird die Abdunkelung - wie an sich bekannt - bis zu dieser Intensität festgelegt.
- Ist die zu schützende Objektfläche nur durch bestimmte Wellenlängen-Bereiche verletzbar, so ist die Reduktion auf diesen Wellenlängenbereichjedoch örtlich spezifisch - eingeschränkt.
- Durch die erfindungsgemäße Aufteilung des Spektralfilters in verschiedene Bereiche lassen sich verschiedene Absorptionsverläufe (Intensitätsreduktion in Funktion der Wellenlänge) erzeugen.
- Durch die räumliche Verschiebbarkeit des Spektralfilters in seiner Anwendungsebene und/oder Anwendungsachse ist der geschützte Bereich in bestimmtem Maße variierbar.
- Durch den erfindungsgemäßen Einsatz dünner Schichten lassen sich beliebige Absorptions-Bilder (Intensitätsreduktion in Funktion der Wellenlänge) des Lichtes bilden.
- Durch den erfindungsgemäßen Einsatz von LCD und/oder elektrochromen Schichten lassen sich elektronisch angesteuert beliebige Absorptionsflächen und/oder Absorptionsverläufe (Intensitätsreduktion in Funktion der Wellenlänge) bilden.

Im obigen Text wird zwar auf ein Operationsmikroskop Bezug genommen; die Erfindung ist jedoch nicht darauf eingeschränkt, sondern steht vielmehr auch anderen Benutzern optischer Geräte mit partieller Reduktion der Beleuchtungs-Intensität und/oder Objekt-Strahlung (z.B. Projektoren, Videound Fotokameras, etc.) offen.

### Figurenbeschreibung

Die Figuren und die Bezugsziffernliste sind - zusammen mit den in den Ansprüchen beschriebenen Merkmalen - integrierender Bestandteil der Offenbarung dieser Anmeldung.

Die Figuren werden zusammenhängend und übergreifend beschrieben. Gleiche Bezugsziffern bedeuten gleiche Bauteile; Bezugsziffern mit unterschiedlichen Indizes geben funktionengleiche Bauteile an.

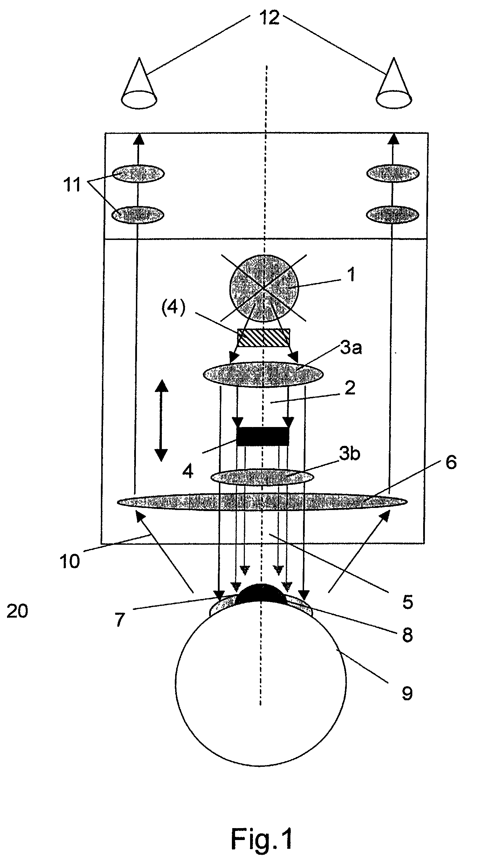

Die Fig.1 zeigt symbolisch den Gesamtaufbau einer optischen Betrachtungseinrichtung, beispielsweise eines Stereo-Operationsmikrokopes ist einer Lichtquelle 1, beispielsweise einer Lampe oder dem Ende eines Lichtwellenleiters, einem Beleuchtungs-Strahlengang 2, einer Beleuchtungs-Optik 3a, 3b, einem erfindungsgemäßen Spektralfilter 4 einem Haupt-Objektiv 6, einem lichtintensitäts-reduzierten Beleuchtungs-Strahlengang 5, beispielsweise zu einem Auge, symbolisch bestehend aus Pupille 7, Iris 8 und Augapfel 9. Zusätzlich werden der Objekt-Strahlengang 10, das Okular 11 sowie der Betrachter 12 symbolisch abgebildet. Der Spektralfilter 4 ist als Variante auch zwischen Lichtquelle 1 und Beleuchtungs-Optik 3 anordenbar.

Die Fig.2 zeigt schematisch das Auge, bestehend aus Pupille 7, Iris 8 und Augapfel 9, in Aufsicht. Es wird der Verlauf der Intensitätsreduktion 24 bei einer bestimmten Wellenlänge Lambda 0 in Funktion des lichtintensitätsreduzierten Beleuchtungsbereiches 21 (x, y) durch den Spektralfilter 4 dargestellt.

Die Fig.3 zeigt einen möglichen Verlauf der Lichtintensitäts-Reduktion in Funktion der Wellenlänge Lambda mit einem bestimmten lichtintensitätsreduzierten Wellenlängenbereich 22 und einer bestimmten Wellenlänge Lambda 0, vgl. die Bezugsziffer 23.

Die Fig.4 zeigt schematisch die elektronische Ansteuerung 25 eines Spektralfilters 4, welcher beispielsweise als elektrochrome Schicht oder als LCD ausgebildet sein kann.

### Funktionsweise

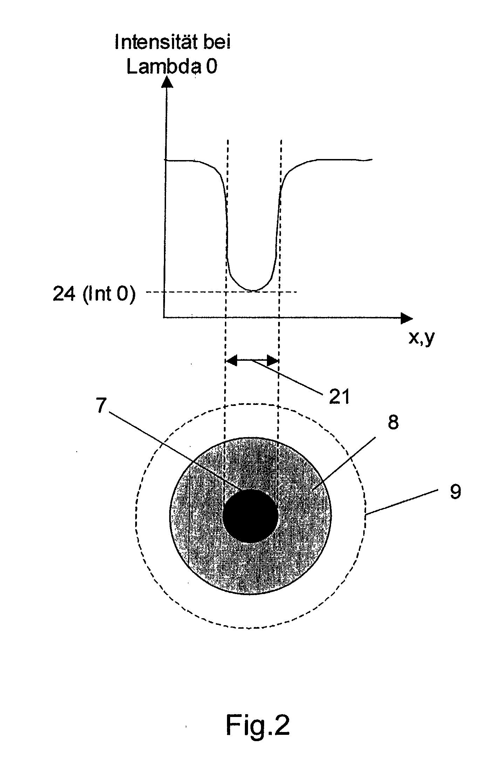

Der von der Lichtquelle 1 ausgehende Beleuchtungs-Strahlengang 2 wird über eine Beleuchtungs-Optik 3a, 3b und über einen dazwischenliegenden Spektralfilter 4 in einem bestimmten räumlichen Bereich 5 lichtwellenlängenabhängig intensitätsreduziert. Die Lage des Filters 4 kann, in Abweichung von der Fig.1, auch zwischen Lichtquelle 1 und Beleuchtungsoptik 3a, 3b sein. Dadurch wird über einen bestimmten lichtempfindlichen räumlichen Objekt-Bereich (x,y), beispielsweise bei einem Auge Retina/Cornea, die Intensität mittels des Spektralfilters 4 reduziert. Der Spektralfilter 4 ist so ausgebildet, dass die Reduktion in einem bestimmten Wellenlängenbereich 21 erfolgt, beispielsweise von 420 nm bis 470 nm, und/oder einen bestimmten Wert beträgt, der beispielsweise zwischen 0% und 90% liegt. Auf den übrigen Beleuchtungsbereich, der nicht abgeschwächt wurde, entfällt die gesamte Lichtleistung und/oder eine wellenlängen-abhängige Lichtleistung. Der Aufbau ermöglicht über die Form des Spektralfilters 4, den lichtreduzierten Beleuchtungsbereich (x, y) beliebig räumlich auszugestalten (Fig.2) und über eine Verschiebung des Spektralfilters und/oder eine Verschiebung der Beleuchtungs-Optik 3a, 3b die räumliche Ausdehnung (x, y) in einem bestimmten Maße zu vergrößern bzw. zu verkleinern.

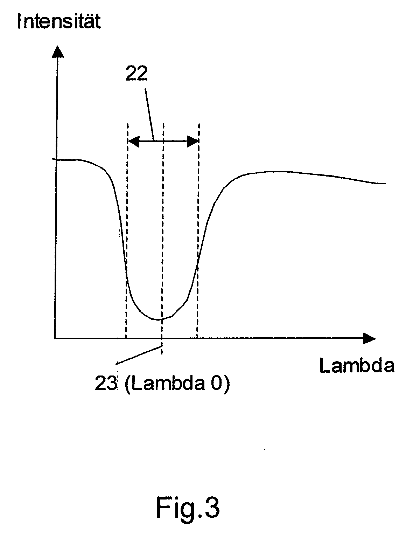

Die Absorptionseigenschaften (Fig.3) des Spektralfilters 4 ermöglichen erfindungsgemäß eine bestimmte Reduktion der Lichtintensität in Funktion der Wellenlänge Lambda, beziehungsweise einen Unterschied der Transparenz in Abhängigkeit vom Ortsbereich des Spektralfilters 4 (Fig.2).

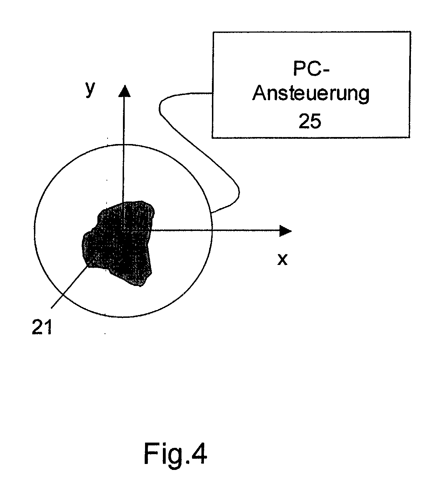

Eine Weiterentwicklung der Erfindung besteht darin, dass der Spektralfilter als LCD und/oder als elektrochrome Schicht ausgebildet ist, welche(s) elektronisch angesteuert 25 ist. Dies ermöglicht, die räumliche Ausdehnung (x,y) und/oder die Intensität und/oder die Wellenlänge des lichtreduzierten Bereiches 21 variabel zu gestalten (Fig.4).

### Bezugsziffernliste

- 1: Lichtquelle (Lampe oder das Ende eines Lichtwellenleiters)
- 2: Beleuchtungs-Strahlengang
- 3, 3a, 3b: Beleuchtungs-Optik
- 4: Spektralfilter
- 5: lichtintensitäts-reduzierter Beleuchtungs-Strahlengang
- 6: Haupt-Objektiv
- 7: Pupille
- 8: Iris
- 9: Augapfel
- 10: Objekt-Strahlengang
- 11: Okular
- 12: Betrachter
- 21: lichtintensitäts-reduzierter Beleuchtungsbereich
- 22: lichtintensitäts-reduzierter Wellenlängenbereich
- 23: Lambda 0
- 24: Intensitätsreduktion (Int 0)
- 25: PC-Ansteuerung der elektrochromen Schicht und/oder des LCD

## Patentansprüche

1. Einrichtung zur partiellen Reduktion der Lichtintensität einer Beleuchtung, beispielsweise in einem Mikroskop mit einem Beleuchtungs-Strahlengang, einem Haupt-Objektiv, einer Lichtquelle und einem Spektralfilter, **dadurch gekennzeichnet, dass** der Spektralfilter (4) so ausgebildet ist, dass er in voneinander unterschiedlichen räumlichen Beleuchtungsbereichen (21) die Lichtintensität in unterschiedlichen Wellenlängenbereichen (22) in bestimmtem Maße (Intensitätsreduktion 0) (24) reduziert.

2. Einrichtung nach Anspruch 1, **dadurch gekennzeichnet, dass** wenigstens ein Bereich des Spektralfilters (4) eine bestimmte Wellenlänge Lambda 0 (23) absorbiert, die beispielsweise im Bereich von 420 bis 470 nm und/oder unterhalb von 400 nm, beziehungsweise im Retina-/Cornea-schädigenden Bereich liegt.

3. Einrichtung nach einem der vorhergehenden Ansprüche, **dadurch gekennzeichnet, dass** die Absorptionskanten wenigstens eines der lichtintensitäts-reduzierten Wellenlängenbereiche (22) flach oder steil ausgebildet sind.

4. Einrichtung nach einem der vorhergehenden Ansprüche, **dadurch gekennzeichnet, dass** der Spektralfilter (4) einen Verlauf aufweist, der von innen nach außen unterschiedliche lichtintensitäts-reduzierte Wellenlängenbereiche (22) hat, beispielsweise innen 420 bis 470 nm um 50% reduziert ist und außen über das gesamte sichtbare Lichtspektrum keine Reduktion aufweist.

5. Einrichtung nach Anspruch 1, **dadurch gekennzeichnet, dass** der Spektralfilter (4) eine bestimmte Lichtintensitäts-Reduzierung Int 0 (24) hat, die beispielsweise zwischen 0 und 90% liegt, insbesondere im Wellenlängenbereich von 420 bis 470 nm.

6. Einrichtung nach Anspruch 1, **dadurch gekennzeichnet, dass** der Spektralfilter (4) einen Verlauf aufweist, der unterschiedliche Lichtintensitäts-Reduzierungen Int 0 (24) in unterschiedlichen Wellenlängenbereichen (22) hat, die beispielsweise innen 90% und außen 0% betragen.

7. Einrichtung nach Anspruch 1, **dadurch gekennzeichnet, dass** der Spektralfilter (4) eine scheibenförmige Form mit variierbarem Lichtintensitäts-reduziertem Beleuchtungsbereich (21) aufweist.

8. Einrichtung nach Anspruch 1, **dadurch gekennzeichnet, dass** der Spektralfilter (4) mechanisch ein- oder ausschwenkbar und/oder -schiebbar ist.

9. Einrichtung nach Anspruch 1, **dadurch gekennzeichnet, dass** der Spektralfilter (4) in seiner Anwendungsebene und/oder Anwendungsachse räumlich verschiebbar ist.

10. Einrichtung nach Anspruch 1, **dadurch gekennzeichnet, dass** der Spektralfilter (4) kippbar angeordnet ist, so dass in einem bestimmten Lichtwellen-Bereich eine Variation der Filtereigenschaften möglich ist.

11. Einrichtung nach einem der vorhergehenden Ansprüche, **dadurch gekennzeichnet, dass** der Spektralfilter (4) so ausgebildet ist, dass durch eine Verschiebung in Axialrichtung separat und/oder in Kombination mit der Beleuchtungs-Optik (1, 3) der lichtintensitäts-reduzierte Beleuchtungsbereich (21) vergrößerbar bzw. verkleinerbar ist.

12. Einrichtung nach einem der einem der vorhergehenden Ansprüche, **dadurch gekennzeichnet, dass** die räumliche Verschiebbarkeit und/oder die Ein- und Ausblendbarkeit des Spektralfilters (4) elektronisch oder manuell ansteuerbar ist.

13. Einrichtung nach einem der vorhergehenden Ansprüche, **dadurch gekennzeichnet, dass** der Spektralfilter (4) als dünne Schicht und/oder als LCD und/oder als elektrochrome Schicht ausgebildet ist.

14. Einrichtung nach einem der vorhergehenden Ansprüche, **dadurch gekennzeichnet, dass** die Intensitätsreduktion Int 0 (24) und/oder die Wellenlänge Lambda 0 (23) und/oder der lichtreduzierte Wellenlängenbereich (22) elektronisch ansteuerbar (25) sind.

15. Einrichtung nach einem der vorhergehenden Ansprüche, **dadurch gekennzeichnet, dass** die räumliche Verschiebung und/oder die Einund Ausblendung des Spektralfilter (4) über einen Regelkreis mit der Abbildung des Objektes koppelbar ist.
